# EUROPEAN PATENT APPLICATION

(11) **EP 1 602 652 A1**
(43) Date of publication of application: **07.12.2005**
(21) Application number: 04716356.3
(22) Date of filing: 02.03.2004
(51) Int. Cl.: C07D 311/62, C07H 17/065, A61K 31/352, A61K 31/7048, A61P 3/04, A61P 3/10, A61P 9/10, A61P 9/12, A61P 43/00, A23L 1/30

(54) **ADIPONECTIN EXPRESSION PROMOTER**

(30) Priority: 03.03.2003 JP 2003055783
(71) Applicant: SAN-EI GEN F.F.I., INC., Toyonaka-shi, Osaka 561-8588 (JP)
(72) Inventor: TSUDA, T., Konseru Yagotootokikiyama 302, Nagoya-shi, Aichi 468-0063 (JP); OSAWA, Toshihiko, Aichi 461-0023 (JP); UENO, Yuki, Gifu-shi, Gifu 500-8848 (JP); AOKI, Hiromitsu, San-ei Gen F.F.I., Inc., Toyonaka-shi, Osaka 561-8588 (JP); UTIDA, Kouji, San-ei Gen F.F.I., Inc., Toyonaka-shi, Osaka 561-8588 (JP); KODA, Takatoshi, San-ei Gen F.F.I., Inc., Toyonaka-shi, Osaka 561-8588 (JP)
(74) Representative: Müller-Boré & Partner Patentanwälte
(86) International application number: PCT/JP2004/002576
(87) International publication number: WO 2004/078741

(57) **Abstract**

To prevent or alleviate various pathologies caused by lifestyle-related diseases, etc., the present invention provides a substance having an effect of promoting the expression and elevating the expression level of adiponectin, and applications thereof. An adiponectin expression promoting agent containing, as an active ingredient, at least one cyanidin compound selected from the group consisting of cyanidin and cyanidin glycosides can be applied to pharmaceutical or food compositions for use in applications in which the pharmacological effects of adiponectin are utilized.

## Description

### TECHNICAL FIELD

The present invention relates to a composition having an effect of promoting adiponectin expression. Specifically, the present invention relates to a composition that can achieve effects of adiponectin, i.e., preventing or alleviating various pathologies, by promoting or increasing the expression of the adiponectin gene of a test subject.

### BACKGROUND OF THE INVENTION

In recent years, caused by a westernized lifestyle such as having a high fat diet, lack of exercise, etc., the increase in so-called lifestyle-related diseases such as diabetes, hypertension, hyperlipidemia, arteriosclerosis, etc., has become an object of public concern in Japan. As a result of intensive research in recent years, it has become clear that such lifestyle-related diseases are caused by genetic predispositions specific to the patient, and various factors relating to internal secretions are involved in the development of pathologies.

Adiponectin is one of the factors relating to internal secretions. Adiponectin, which was originally separated from human adipose tissue by Maeda et al. in 1996, is a secreted protein of about 30 KDa that is specifically expressed in animal adipose tissues and is composed of 244 amino acid residues (K. Maeda et al., *Biochem. Biophys. Res. Commun.,* 221, (1996): 286-289). Since then, the effects of adiponectin have been gradually revealed by research conducted by several groups. Specifically, anti-obesity, anti-diabetes and anti-arteriosclerosis effects, active oxygen generation reduction, etc., are known (A. H. Berg et al., *Nat. Med.,* 7 (2001): 947-953); T. Yamauchi et al., *Nat. Med.,* 8, (2002): 1288-1295; Iichiro Shimomura et al., *Medical Science Digest,* 28(12), (2002): 17-20; Toshimasa Yamauchi, Takashi Kadowaki, *Experimental Medicine,* 20 (12) (August), (2002): 1762-1767; and H. Motoshima et al., *Biochemical and Biophysical Research Communications* 315 (2004): 264-271).

The above document (Experimental Medicine) discloses that an anti-arteriosclerosis effect is achieved by an action of adiponectin secreted from adipose tissue, i.e., suppressing adhesion of a monocyte to a vascular endothelical cell, preventing macrophages from accumulating lipid therein and becoming a foam cell, and furthermore suppressing growth and migration of smooth muscle cells.

It is known that adiponectin is secreted from adipose cells after a meal, promotes oxidation of fatty acids by activating AMP-activated kinase (AMPK), reduces blood-sugar levels by burning excess fat and sucrose after a meal. However, if fat is accumulated in muscles, liver, etc., due to obesity or the like, the ability of adipose cells to secrete adiponectin decreases, and absorption of sugars in the blood decreases, leading to the development of diabetes. In other words, adiposis such as obesity, hyperlipidemia, etc., closely relates to diabetes through the effects of adiponectin.

As described above, the pharmacological effects of adiponectin are being gradually revealed, and accordingly several projects have been progressing aiming to apply adiponectin per se or its gene to a medicine in order to utilize the excellent medicinal actions of adiponectin in the medical field.

Cyanidin and glycosides thereof are known as components contained in an anthocyanin-based colorant extracted from plants. It is known that anthocyanin has anti-oxidant, antimutagenic, antitumor, and antiulcer actions, and effects of increasing vascular flow, improving visual performance, suppressing hypertension, and reducing liver dysfunction (for example, *Anthocyanin, color of food and health,* Kenpakusha, May 10, 2000).

With respect to cyanidin glycosides, Japanese Unexamined Patent Publication No. 2000-178295 discloses that cyanidin glycosides have an antitumor effect. The publication discloses that it is preferable to intake foods comprising cyanidin glycosides as an active ingredient to prevent or alleviate diseases such as hyperlipidemia, arteriosclerosis, diabetes, fatty liver, myocardial infarction, etc. However, among these effects, only the antitumor effect is objectively demonstrated by disclosed experimental data, and the publication nowhere discloses pharmacological data regarding anti-obesity or anti-diabetes effects. Furthermore, from the medical point of view, it is scarcely believable that the antitumor effect can alleviate obesity and the other diseases mentioned above.

### BRIEF DESCRIPTION OF THE DRAWINGS

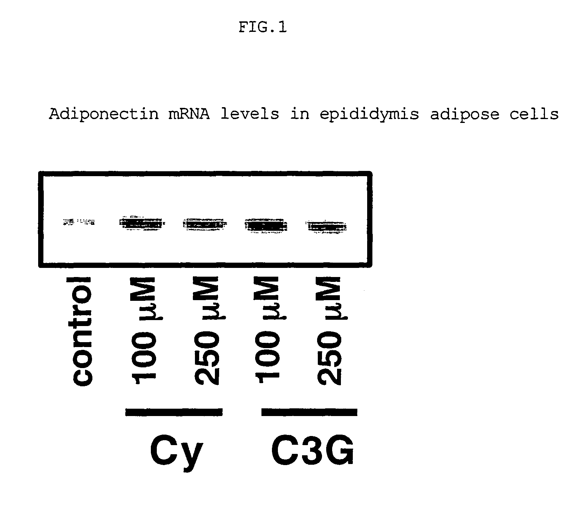

Fig. 1 is a diagram wherein the adiponectin mRNA levels in the epididymis adipose cells of Experimental Example 1 that were cultured after the addition of a cyanidin compound (either cyanidin (Cy) or cyanidin glycoside (cyanidin 3-*O*-β-D-glucoside, hereinafter, this may be simply referred to as "cyanidin 3-glucoside" (C3G) or "cyanidin 3-*O*-glucoside") is compared to the adiponectin mRNA levels in the epididymis adipose cells that were cultured after the addition of DMSO instead of the cyanidin compound (control).

### DISCLOSURE OF THE INVENTION

As described above, in recent years, with respect to adiponectin that is specifically preset in adipose tissue, its excellent medicinal effects such as preventing or alleviating of hyperlipidemia, arteriosclerosis, diabetes or the like lifestyle-related diseases have gradually been elucidated. Therefore, not only adiponectin per se, but also substances that can increase the effects of adiponectin or promote the expression or secretion of adiponectin in vivo are believed to be effective for preventing or alleviating various pathologies including lifestyle-related diseases, attributable to the pharmacological effects of adiponectin.

The present invention aims to provide, based on the above-described knowledge, substances having an effect of promoting and increasing the expression of adiponectin, and the applications of such substances.

Under these circumstances, the present inventors have conducted extensive research and found that cyanidin and cyanidin glycosides have an effect of promoting the expression of mRNA of adiponectin, and the above objects can be achieved by using cyanidin and such cyanidin glycosides. The present invention was accomplished based on these findings.

In other words, the present invention encompasses the following subject matter.
1. An adiponectin expression promoting agent comprising as an active ingredient at least one cyanidin compound selected from the group consisting of cyanidin and cyanidin glycosides.
2. An adiponectin expression promoting agent according to Item 1, wherein the cyanidin glycoside is as shown in General Formula (1) below: 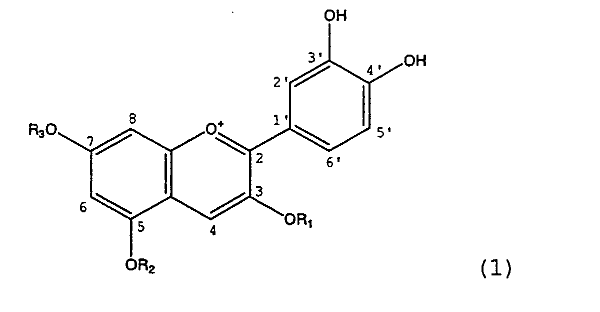 wherein R₁ is a sugar to which an organic acid may be linked, and R₂ and R₃, which may be the same or different, are sugar to which at least one hydrogen atom or organic acid may be linked.
3. An adiponectin expression promoting agent according to Item 2, wherein the cyanidin glycoside represented by General Formula (1) comprises as the sugar β-D-glucose, β-D-galactose, α-L-rhamnose, β-D-xylose, β-D-arabinose, β-D-glucuronic acid, rutinose, sophorose, gentiobiose, Sambubiose, rhamnose, laminaribiose, gentiotriose, robinobiose, 2^{G}-glucosylrutinose, or 2^{G}-xylosylrutinose.
4. An adiponectin expression promoting agent according to Item 2, wherein the cyanidin glycoside represented by General Formula (1) comprises, as the sugar, a sugar to which at least one organic acid selected from the group consisting of p-coumaric acid, caffeic acid, ferulic acid, sinapic acid, p-hydroxybenzoic acid, gallic acid, acetic acid, oxalic acid, malonic acid, succinic acid, and malic acid is linked.
5. An adiponectin expression promoting agent according to Item 2, wherein the cyanidin glycoside represented by General Formula (1) is a cyanidin compound to which a β-D-glucose is directly linked at the 3-position through an oxygen atom with proviso that the β-D-glucose optionally having an organic acid or a further sugar linked thereto.
6. An adiponectin expression promoting agent according to Item 2, wherein the cyanidin glycoside represented by General Formula (1) is cyanidin 3-*O*-β-D-glucoside.
7. An adiponectin expression promoting agent according to Item 1, wherein the cyanidin compound is of plant origin.
8. An adiponectin expression promoting agent according to Item 7, wherein the plant is a purple sweet potato, red cabbage, purple corn, elderberry, strawberry, boysenberry, raspberry, cranberry, blackberry, blueberry, perilla, red-kerneled rice, black-kerneled rice, black soybean, grape, or hibiscus.
9. A pharmaceutical or food composition which is used for an application in which a pharmacological effect of adiponectin is utilized, the pharmaceutical or food composition comprising as an active ingredient at least one cyanidin compound selected from the group consisting of cyanidin and cyanidin glycosides, with the proviso that when the cyanidin compound is cyanidin 3-*O*-β-D-glucoside, a use for anti-obesity or anti-diabetes is excluded.
10. A pharmaceutical or food composition according to Item 9, wherein the cyanidin glycoside is a cyanidin compound as shown in General Formula (1) below: 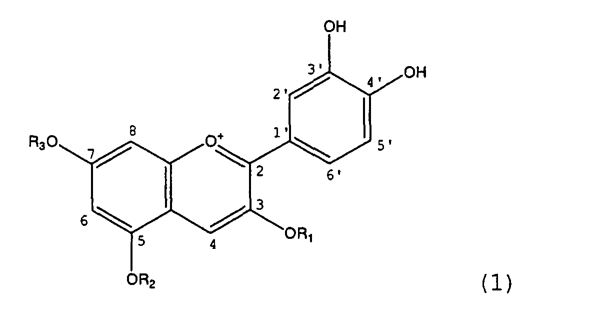 wherein R₁ is a sugar to which an organic acid may be linked, and R₂ and R₃, which may be the same or different, are sugars to which at least one hydrogen atom or organic acid may be linked.
11. A pharmaceutical or food composition according to Item 10, wherein the cyanidin glycoside represented by General Formula (1) comprises as the sugar β-D-glucose, β-D-galactose, α-L-rhamnose, β-D-xylose, β-D-arabinose, β-D-glucuronic acid, rutinose, sophorose, gentiobiose, Sambubiose, rhamnose, laminaribiose, gentiotriose, robinobiose, 2^{G}-glucosylrutinose, or 2^{G}-xylosylrutinose.
12. A pharmaceutical or food composition according to Item 10, wherein the cyanidin glycoside represented by General Formula (1) comprises, as the sugar, a sugar to which at least one organic acid selected from the group consisting of p-coumaric acid, caffeic acid, ferulic acid, sinapic acid, p-hydroxybenzoic acid, gallic acid, acetic acid, oxalic acid, malonic acid, succinic acid, and malic acid is linked.
13. A pharmaceutical or food composition according to Item 10, wherein the cyanidin glycoside represented by General Formula (1) is a cyanidin compound to which a β-D-glucose is directly linked at the 3-position through an oxygen atom with proviso that the β-D-glucose optionally having an organic acid or a further sugar linked thereto.
14. A pharmaceutical or food composition according to Item 10, wherein the cyanidin glycoside represented by General Formula (1) is cyanidin 3-*O*-β-D-glucoside.
15. A pharmaceutical or food composition according to Item 9, wherein the cyanidin compound is of plant origin.
16. A pharmaceutical or food composition according to Item 15, wherein the plant is a purple sweet potato, red cabbage, purple corn, elderberry, strawberry, boysenberry, raspberry, cranberry, blackberry, blueberry, perilla, red-kerneled rice, black-kerneled rice, black soybean, grape, or hibiscus.
17. A pharmaceutical or food composition according to Item 9, wherein the pharmacological effect of adiponectin is to alleviate a lifestyle-related disease, and the pharmaceutical or food composition is used for preventing or alleviating a lifestyle-induced disease.
18. A pharmaceutical or food composition according to Item 9, wherein the adiponectin has at least one pharmacological effect selected from the group consisting of anti-obesity, anti-diabetes, anti-arteriosclerosis, and active oxygen generation reduction, and the pharmaceutical or food composition is used for preventing or alleviating obesity, diabetes, arteriosclerosis, hypertension, or at least one pathology due to active oxygen.

The present invention is explained below.

### (1) Adiponectin expression promoting agent

The present invention relates to an adiponectin expression promoting agent comprising cyanidin or a cyanidin glycoside (hereunder, cyanidin and cyanidin glycosides may be referred to as a "cyanidin compound" in the present specification) as an active ingredient.

In the present invention, "adiponectin expression promoting agent" means a substance that can increase the expression of an adiponectin gene (i.e., expression of mRNA). Note that adiponectin is a protein that is inherently expressed specifically in animal adipose tissues (adipose cells). Therefore, it is preferable that the adiponectin expression promoting agent of the present invention promote or increase expression of adiponectin in adipose tissues (adipose cells); however, it is not limited to adiponectin expression in adipose tissues (adipose cells), and substances that can be used for promoting and increasing the expression of the adiponectin gene in general are encompassed within the scope of the adiponectin expression promoting agent of the present invention. As long as the adiponectin expression promoting agent is encompassed by the above, the adiponectin expression promoting agent of the present invention may comprise cyanidin or a glycoside thereof, or a composition containing the same. There is no limitation to the form of the promoting agent, purpose of its use, and application thereof.

Cyanidin used as an active ingredient of the adiponectin expression promoting agent is a compound represented by the formula below:

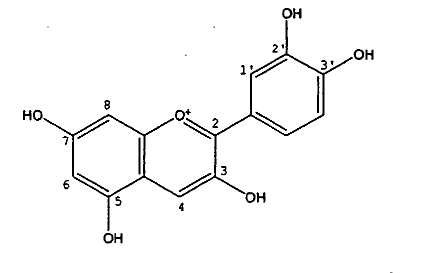

Cyanidin is widely present in plants as an aglycon in anthocyanidin glycoside colorant (anthocyanin-based colorant).

Cyanidin glycosides, which are used as an active ingredient of the adiponectin expression promoting agent in the same manner as cyanidin, have a structure in which at least one sugar is linked to the hydroxy group at the 3-position of the cyanidin, and are widely present in plants as a coloring component of anthocyanin colorant in the same manner as cyanidin.

A specific example of a cyanidin glycoside is a compound represented by General Formula (1) as below: 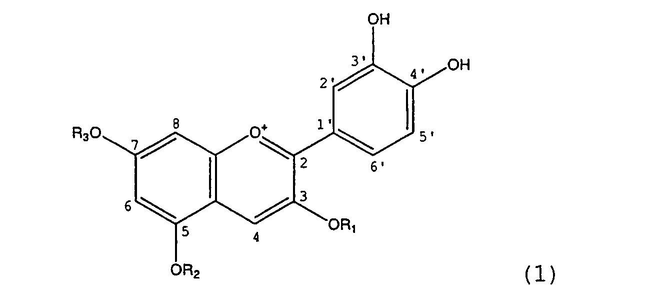 wherein R₁ that is linked to the 3-position of the cyanidin through an oxygen atom is a sugar; R₂ and R₃ linked to the 5- and 7-positions of the cyanidin through oxygen atoms are both hydrogen atoms or sugars, or one is a hydrogen atom and the other is a sugar.

Here, the sugar that is linked to the cyanidin through an oxygen atom may be a monosaccharide, disaccharide or trisaccharide. Specific examples of monosaccharides are β-D-glucose, β-D-galactose, α-L-rhamnose, β-D-xylose, β-D-arabinose, and β-D-glucuronic acid. Specific examples of disaccharides are rutinose, sophorose, gentiobiose, Sambubiose, rhamnose, and laminaribiose. Specific examples of trisaccharides are gentiotriose, robinobiose, 2^{G}-glucosylrutinose, and 2^{G}-xylosylrutinose.

A preferable example of a sugar that is linked to the 3-position of the cyanidin through the oxygen atom is β-D-glucose. For example, cyanidin 3-*O*-β-D-glucoside, which is an example of a cyanidin glycoside wherein a monosaccharide(glucose) is singly linked to the 3-position of the cyanidin by a glucoside linkage through an oxygen atom, is shown by the formula below:

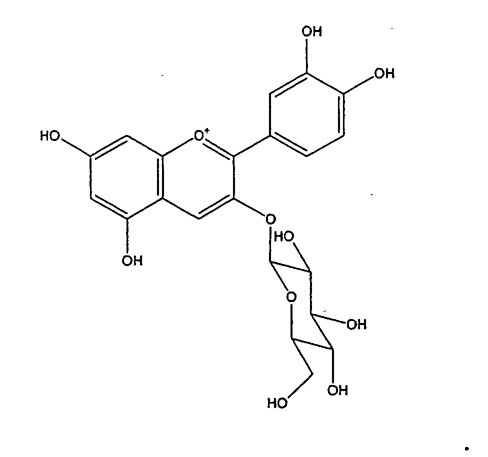

Examples of cyanidin glycosides wherein a disaccharide is linked to the 3-position of cyanidin by a glucoside linkage through an oxygen atom include cyanidin 3-sophoroside(3-*O*-(2-*O*-(β-D-glucopyranosyl)-β-D-glucopyranosyl) cyanidin) represented by the formula below: 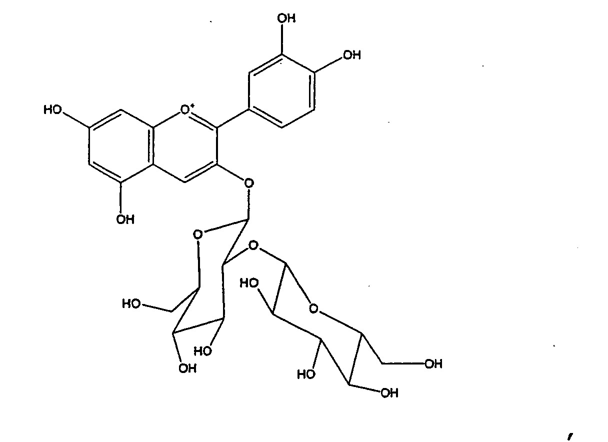 cyanidin 3-Sambubioside (3-*O*-(2-*O*-(β-D-xylopyranosyl)-β-D-glucopyranosyl) cyanidin) represented by the formula below: 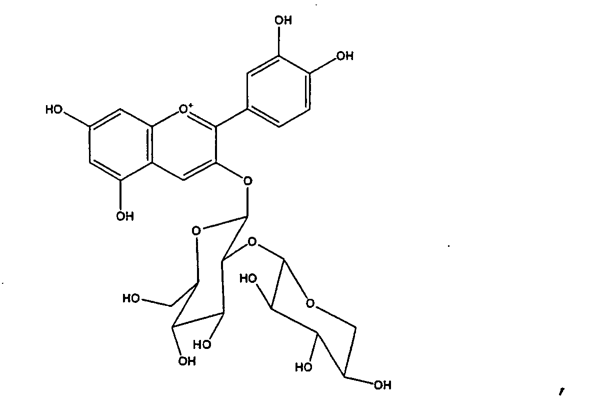 and cyanidin 3-*O*-rutinoside (3-*O*-(6-*O*-(α-L-rhamnosyl)-β-D-glucopyranosyl) cyanidin) represented by the formula below:

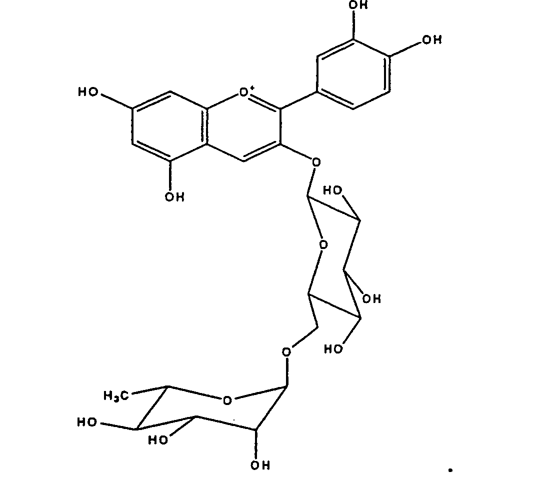

Note that the sugar linked to the cyanidin may be such that one or more organic acids are linked to the above-mentioned various sugars, mainly at the 6-position hydroxy group, by ester bonding. Examples of such organic acids are aromatic organic acids such as p-coumaric acid, caffeic acid, ferulic acid, sinapic acid and like hydroxy cinnamic acids; p-hydroxybenzoic acid, gallic acid and like hydroxybenzoic acids; and acetic acid, oxalic acid, malonic acid, succinic acid, malic acid and like aliphatic organic acids; etc.

A specific example thereof is the cyanidin glycoside (3-*O*-(6-*O*-malonyl-β-D-glucopyranosyl) cyanidin) shown in the following formula, wherein glucose to which malonic acid is linked at its 6-position by an ester linkage is linked to the 3-position of the cyanidin through an oxygen atom by glucoside linkage:

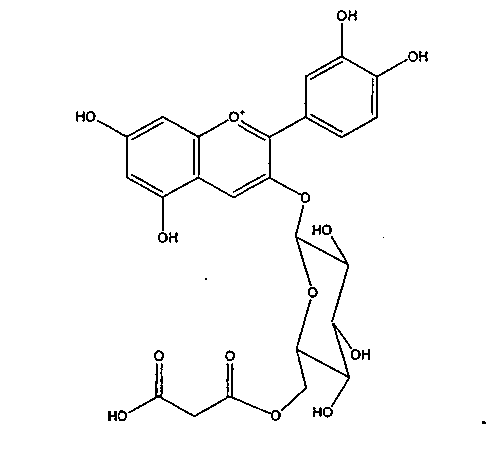

The linkage site of the sugar(s) is not limited to the 3-position of the cyanidin and may be one or more positions other than the 3-position. Examples of the linkage site are the 5-position, 7-position, 3- and 5-positions, 3- and 7-positions, 5-and 7-positions, or 3-, 5- and 7-positions of the cyanidin. An example of a cyanidin glycoside in which sugars are linked to a plurality of positions of the cyanidin is a cyanidin compound represented by the formula below:

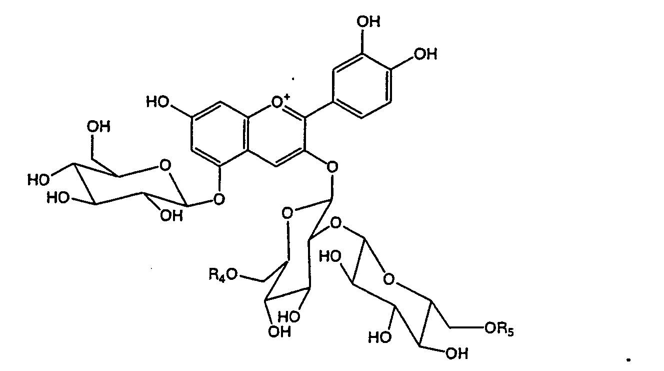

Here, both R₄ and R₅ may be hydrogen atoms (in this case, the cyanidin compound is cyanidin 3-sophoroside-5-glucoside) or organic acid groups, or one may be a hydrogen atom and the other may be an organic acid group. A specific example wherein both R₄ and R₅ are organic acid groups is a cyanidin compound (3-*O*-(2-*O*-(6-*O*-(E)-ferulyl-β-D-glucopyranosyl)-6-*O*-(E)-caffeyl-β-D-glucopyranosyl)-5-*O*-(β-D-glucopyranosyl) cyanidin) in which R₄ is a caffeic acid group and R₅ is a ferulic acid group as represented by the formula below:

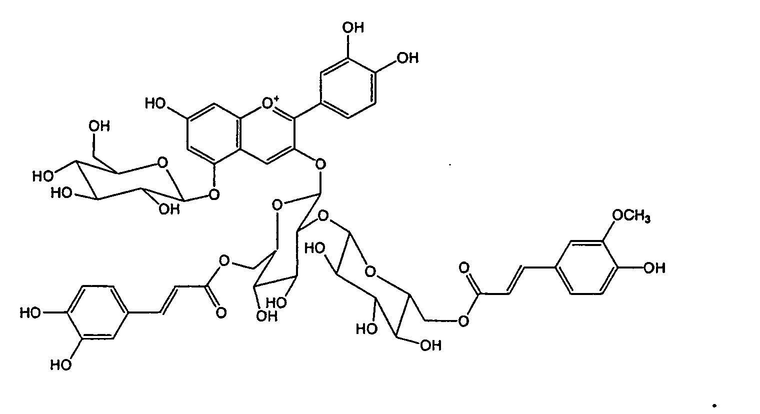

Note that such cyanidin compounds are present in plants such as purple sweet potato (*Ipomoea batatas Poir*); red cabbage (*Brassica oleracea L. var. capitata L.*); purple corn (*Zea mays L.*); elderberries (*Sambucus caerulea Rafin., S. canadensis L., S. nigra L.*), strawberry (*Fra garia X ananassa Duchesne*), boysenberry (*Rubus strigoosus Michx.*), raspberries (*Rubus idaeus L.*(redraspberry) and *R. fruticosus L.* (blackberry), cranberry (*Vaccinium macrocarpon Ait.*), blueberries (e.g., *Vaccinium corymbosum L.*(*highbushblueberry*)*, V.ashei Reade* (rabbiteye blueberry), *V. myrtillus L.* (whortleberry), etc.) and like berries; perilla *(Perilla ocimoides* (frutescence) *L. var crispa*)*;* red-kerneled rice (*Oryza sativa L*.), black-kerneled rice (*Oryza sativa L*.), black beans (e.g., soybean (*Glucine max Merrill*), kidney bean (*Phaseolus vulagaris L.*), green pea (*Pisum sativum L.*), adzuki bean (*Pisum sativum L.*), black eye bean (*Vigna sinensis Savi*), etc.) and like grains; grapes (*Vitis vinifera* *L.* (European origins), and *V.labrusca L.* (American origins)); hibiscus (*Hibiscus sabdariffa L. var sabdariffa*)*,* etc.

For example, tuberous roots of purple sweet potatoes contain the cyanidin compounds that are represented by the formula below and listed in Table 1:

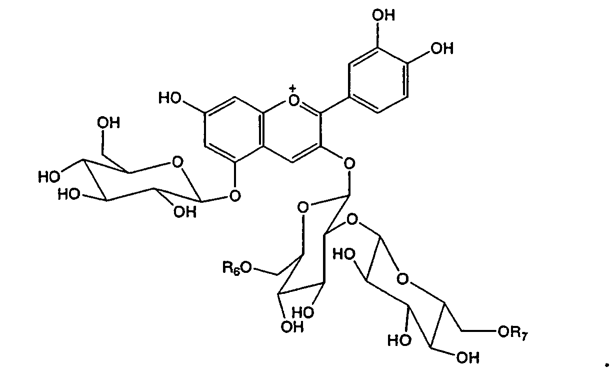

**<Table 1>**

| Compound | R₆ | R₇ | Compound name |
|---|---|---|---|
| PSP-1 | H | H | 3-*O*-(2-*O*-β-D-glucopyranosyl-β-D-glucopyranosyl)-5-*O*-(β-D-glucopyranosyl) cyanidin |
| PSP-2 | Caf | H | 3-*O*- (6-*O*-(*E*)-caffeyl-2-*O*-β-D-glucopyranosyl-β -D-glucopyranosyl)-5-*O*-(β-D-glucopyranosyl) cyaniding |
| PSP-3 | Caf | Caf | 3-*O*-(2-*O*-(6-*O*-(*E*)-caffeyl-β-D-glucopyranosyl)-6-*O*-(*E*)-caffeyl-β-D-glucopyranosyl)-5-*O*-(β-D-glucopyranosyl) cyanidin |
| PSP-4 | Caf | pHB | 3-*O*-(2-*O*-(6-*O*-*p*-hydroxybenzoyl-β-D-glucopyranosyl)-6-*O*-(*E*)-caffeyl-β-D-glucopyranosyl)-5-*O*-(β-D-glucopyranosyl) cyanidin |
| PSP-5 | Caf | Fer | 3-*O*-(2-*O*-(6-*O*-(*E*)-ferulyl-β-D-glucopyranosyl)-6-*O*-(*E*)-caffeyl-β-D-glucopyranosyl)-5-*O*-(β-D-glucopyranosyl) cyanidin |

In Table 1, "H" stands for a hydrogen atom, "Caf" stands for caffeic acid, "pHB" stands for p-hydroxybenzoic acid, and "Fer" stands for ferulic acid. "PSP" in the table is an abbreviation for "purple sweet potato" used by the present inventors for the sake of convenience.

Red cabbage leaves contain the cyanidin compounds represented by the formula below and listed in Table 2:

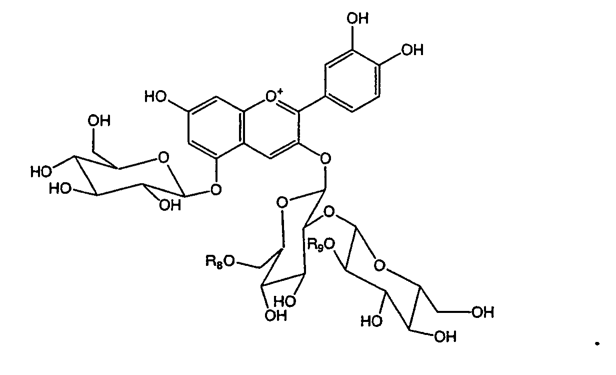

**<Table 2>**

| Compound | R₈ | R₉ | Compound name |
|---|---|---|---|
| RC-1 | H | H | 3-*O*-(2-*O*-(β-D-glucopyranosyl)-β-D-glucopyranosyl)-5-*O*-(β-D-glucopyranosyl) cyanidin |
| RC-2 | Sin-Glc | H | 3-*O*- (6-*O*- (4-*O*- (β-D-glucopyranosyl)-(*E*)-sinapyl)-2-*O*-(β-D-glucopyranosyl)-β-D-glucopyranosyl)-5-*O*-(β-D-glucopyranosyl) cyanidin |
| RC-3 | PC | H | 3-*O*-(6-*O*-*p*-cumaryl-2-*O*-(β-D-glucopyranosyl)-β-D-glucopyranosyl)-5-*O*-(β-D-glucopyranosyl) cyanidin |
| RC-4 | Fer | H | 3-*O*-(6-*O*-(*E*)-ferulyl-2-*O*-(β-D-glucopyranosyl)-β-D-glucopyranosyl)-5-*O*-(β-D-glucopyranosyl) cyanidin |
| RC-5 | Sin | H | 3-*O*-(6-*O*-(*E*)-sinapyl-2-*O*-(β-D-glucopyranosyl)-β-D-glucopyranosyl)-5-*O*-(β-D-glucopyranosyl) cyanidin |
| RC-6 | pC | Sin | 3-*O*- (6-*O*-(*E*)-*p*-coumaryl-2-*O*-(2-*O*-(*E*)-sinapyl-β-D-glucopyranosyl)-β -D-glucopyranosyl)-5-*O*-(β-D-glucopyranosyl) cyanidin |
| RC-7 | Fer | Sin | 3-*O*-(6-*O*-(*E*)-ferulyl-2-*O*-(2-*O*-(*E*)-sinapyl-β-D-glucopyranosyl)-β-D-glucopyranosyl)-5-*O*-(β-D-glucopyranosyl) cyanidin |
| RC-8 | Sin | Sin | 3-*O*-(6-*O*-(*E*)-sinapyl-2-*O*-(2-*O*-(*E*)-sinapyl-β-D-glucopyranosyl)-β-D-glucopyranosyl)-5-*O*-(β-D-glucopyranosyl) cyanidin |

In the table, "H" stands for a hydrogen atom, "Sin" stands for sinapic acid, "pC" stands for p-coumaric acid, "Fer" stands for ferulic acid, and "Sin-Glc" stands for a glucose to which a sinapic acid is linked. "RC" in the table is an abbreviation for "Red Cabbage (red cabbage)" used by the present inventors for the sake of convenience.

Perilla leaves contain the cyanidin compounds represented by the formula below and listed in Table 3:

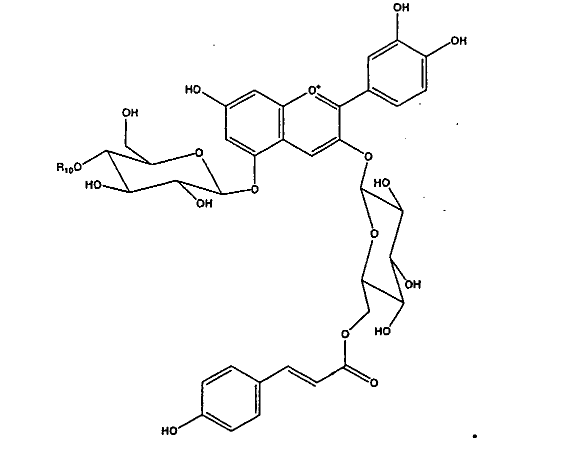

**<Table 3>**

| Compound | R₁₀ | Compound name |
|---|---|---|
| Shisonin | H | 3-*O*-(6-*O*-(*E*)-*p*-coumaryl-β-D-glucopyranosyl)-5-*O*-(β-D-glucopyranosyl) cyanidin |
| Malonylshisonin | Ma | 3-*O*-(6-*O*-(*E*)-*p*-coumaryl-β-D-glucopyranosyl)-5-*O*-(6-*O*-malonyl-β-D-glucopyranosyl) cyanidin |

In Table 3, "H" stands for a hydrogen atom, and "Ma" stands for malonic acid. "Shisonin" and "Malonylshisonin" in the table are names of the main anthocyanins contained in perilla.

Elderberry fruits contain the cyanidin compounds represented by the formula below and listed in Table 4:

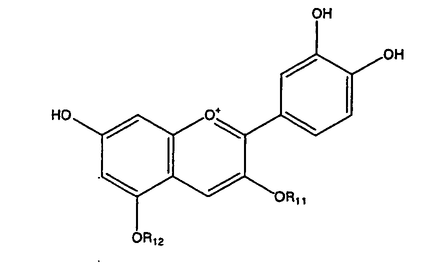

**<Table 4>**

| Compound | R₁₁ | R₁₂ | Compound name |
|---|---|---|---|
| EB-1 | Xyl-Glc | Glc | 3-*O*-(2-*O*-(β-D-xylopyranosyl)-β-D-glucopyranosyl)-5-*O*-(β-D-glucopyranosyl) cyanidin |
| EB-2 | Glc | Glc | 3-*O*-(β-D-glucopyranosyl)-5-*O*- (β-D-glucopyranosyl) cyanidin |
| EB-3 | Xyl-Glc | H | 3-*O*-(2-*O*-(β-D-xylopyranosyl)-β-D-glucopyranosyl) cyanidin |
| EB-4 | Glc | H | 3-*O*-(β-D-glucopyranosyl) cyanidin |

In Table 4, "H" stands for a hydrogen atom, "Glc" stands for glucose, and "Xyl" stands for xylose. "EB" in the table is an abbreviation for "Elderberry (elderberry)" used by the present inventors for the sake of convenience.

The seeds and core of purple corns, skin of grapes, and petals and sepals of hibiscus contain 3-O-β-D-glucopyranosyl cyanidin.

The adiponectin expression promoting agent of the present invention may contain the above-mentioned cyanidin and cyanidin glycosides singly or in any combination of two or more such cyanidin glycosides as an active ingredient. While there is no limitation, the preferable cyanidin glycoside is, however, cyanidin 3-*O*-β-D-glucoside (cyanidin 3-*O*-β-D-glucoside).

Some of these cyanidin and cyanidin glycosides may be obtained by semisynthetic or synthetic methods known in the art; however, they usually can be obtained from plants. Note that cyanidin, cyanidin 3-*O*-β-D-glucoside, cyanidin 3,5-di-*O*-glucoside, and cyanidin 3-*O*-rutinoside can be obtained commercially.

When cyanidin and cyanidin glycosides are obtained from plants, the material plants are not limited as long as they contain cyanidin or cyanidin glycoside. Examples of usable plants include the above-mentioned purple sweet potato; red cabbage; purple corn; elderberry, strawberry, boysenberry, raspberry, cranberry, blackberry, blueberry and like berries; perilla; red-kerneled rice, black-kerneled rice, black soybean and like grains; grapes; hibiscus, etc.

To obtain cyanidin or a cyanidin glycoside, tuberous roots, stalks, leaves, roots, petals, fruits or seeds of the plants, etc., which are the parts that contain cyanidin or cyanidin glycosides, are usually used; however, the plant as a whole may be used. It is also possible to use cultured cells of tissue that contains cyanidin or a cyanidin glycoside. The plant parts which contain cyanidin or cyanidin glycosides are, for example, the seeds and cores of purple corns, leaves of red cabbage and perilla, tuberous roots of purple sweet potatoes, fruits of berries, skin and juice of grapes, seeds of rice and beans, and petals and sepals of hibiscus.

The method for obtaining cyanidin or a cyanidin glycoside (cyanidin compound) from plants is not limited, and a plant extract that contains a cyanidin compound is usually subjected to one or more conventional purification methods suitably selected from adsorption treatment, ion exchange treatment, pH adjustment treatment, extraction treatment, membrane-separation treatment, salt precipitation treatment, etc.

The method for obtaining a plant extract that contains a cyanidin compound is, for example, dipping the above-mentioned material plants in an acidic aqueous solvent obtained by using, for example, sulfuric acid, hydrochloric acid, phosphoric acid, nitric acid or a like inorganic acid, or citric acid, malic acid, acetic acid, lactic acid or a like organic acid in such a manner that its pH is in the range from about 1 to 5, and preferably about 1 to 4, to extract a fraction that contains a cyanidin compound. Here, the plants may be used as they are, or after having been cut or crushed into a desired size. It is also possible to use dried plants in such various forms.

Examples of aqueous solvents used for extraction are water, and a mixture of water and an organic solvent(s) compatible with water. Preferable examples of such organic solvents are methanol, ethanol, propanol, isopropanol, butanol, hexanol, heptanol and like C₁₋₆, and preferably C₁₋₄ lower alcohols. Water and a mixture of water and ethanol (aqueous ethanol) are particularly preferable.

The extraction method is not limited, and it is possible to employ a percolation method or a method such that the material plant is soaked in the above-described aqueous solvent overnight or for several to several tens of hours (for example, 4 to 12 hours).

Solid components in the thus-obtained extract are removed by a conventional solid-liquid-separation method such as filtration, coprecipitation, centrifugation, etc., and the filtrate (or supernatant) is collected as a cyanidin compound-containing plant extract (water-soluble extract).

The thus-obtained plant extract contains the cyanidin compound (cyanidin and/or cyanidin glycosides) as well as impurities. Therefore, after being concentrated if necessary, the plant extract is subjected to a conventional purification treatment such as adsorption treatment, ion exchange treatment, membrane-separation treatment, pH adjustment treatment, extraction treatment, salt precipitation treatment or the like. Such purification treatments may be combined in any combination, or a single treatment may be repeated under the same or different conditions.

Examples of adsorption treatments are those conducted using activated carbon, silica gel, porous ceramics or the like, and adsorption treatments conducted using various synthetic resins such as nonionic absorption resins, etc.

When the extract is purified using a nonionic absorption resin, it is possible to use, for example, styrene-based Duolite S-861 (trade mark: Duolite, manufactured by Diamond Shamrock Corporation (U.S.A.), the same holds true for the following products named Duolite), Duolite S-862, Duolite S-863 or Duolite S-866; aromatic based Sepabeads SP70 (trade mark: manufactured by Mitsubishi Chemical Corporation, the same holds true for the following products named Sepabeads), Sepabeads SP700, Sepabeads SP825, Sepabeads SP207; Diaion HP10(trade mark: manufactured by Mitsubishi Chemical Corporation, the same holds true for the following products named Diaion), Diaion HP20, Diaion HP21, Diaion HP40, or Diaion HP50; or Amberlight XAD-4(trade mark: product of Organo Corporation, the same holds true for the following products named Amberlight), Amberlight XAD-7, or Amberlight XAD-2000.

Ion exchange treatment can be conducted by following a conventional method using a standard resin such as a cation exchange resin, anion exchange resin, etc. For example, it is possible to use, as a cation exchange resin, Diaion SK 1B (trade mark: manufactured by Mitsubishi Chemical Corporation, the same holds true for the following products named Diaion), Diaion SK 102, Diaion SK 116, Diaion PK 208, Diaion WK10, Diaion WK20, etc., and as an anion exchange resin, Diaion SA 10A (trade mark: manufactured by Mitsubishi Chemical Corporation, the same holds true for the following products named Diaion), Diaion SA 12A, Diaion SA 20A, Diaion PA 306, Diaion WA 10, Diaion WA 20, etc.

Membrane-separation treatment widely encompasses various separation methods using membranes, for example, filtration treatment using a functional polymer membrane such as membrane filter, ultrafiltration membrane, reverse osmosis membrane, electrodialysis membrane, etc.

A pH adjustment treatment can be conducted by exposing a plant extract or a treated liquid that has been subjected to the above-mentioned various treatments to a suitable pH condition. Preferable pH adjustment treatment is an acid treatment wherein the extract or treated liquid is exposed to an acidic condition of pH 1 to pH 4, and preferably pH 1 to pH 3.

An example of extraction treatment is such that a plant extract or treated liquid that has been subjected to the above-mentioned various treatments is made to contact carbon dioxide, ethylene, propane or the like in a closed system whose temperature and pressure are at critical point or higher.

Note that a method for analyzing cyanidin compounds contained in plants, and for determining the structure of the isolated cyanidin compounds has already been established (see, for example, *New edition, Natural edible colorant,* Korin, March 22, 2001: 97-128). Therefore, purification and isolation of a cyanidin compound from the above-mentioned plants can be conducted and confirmed by employing such known analytical methods in combination with mass spectroscopy (MS), NMR, and/or methods for analyzing sugars after hydrolysis. Furthermore, among cyanidin compounds, cyanidin, cyanidin 3-*O*-β-D-glucoside, cyanidin 3,5-di-*O*-glucoside, and cyanidin 3-*O*-rutinoside are commercially available as single products as described above, and therefore it is also possible to conduct purification and isolation using these products as reference samples or standards.

The active ingredient of the adiponectin expression promoting agent of the present invention does not necessarily have to be a purified cyanidin compound, and within a range that does not adversely affect the effects of the invention, partially purified cyanidin compound may be used. Examples of partially purified cyanidin compounds are the above-mentioned plant extracts that contain a cyanidin compound, and purified fractions thereof.

All that is necessary is that the adiponectin expression promoting agent of the present invention contains as an active ingredient at least one member selected from the group consisting of the above-mentioned cyanidin and cyanidin glycosides, and the scope of the invention is not limited by the form or application of the adiponectin expression promoting agent. Preferably, the adiponectin expression promoting agent of the present invention is used for the purpose of being orally or parenterally administered to a human or animal, and is in a form suitable for its administration route. Note that the content of the cyanidin compound (total amount) contained in the promoting agent may be suitably selected from the range of 0.02 to 100 wt% per 100 wt% of the adiponectin expression promoting agent, depending on the application and/or administration route.

The form of the adiponectin expression promoting agent is not limited as long as it can be administered to a test subject. Specific examples thereof are powders, tablets, emulsions, capsules, granules, chewables, solutions, syrups and like forms suitable for oral administration; and injections, drops, suppositories, patches, creams, ointments, lotions (solutions) and like forms suitable for parenteral administration. The adiponectin expression promoting agent can be formed into these forms by following conventional methods using solid or liquid excipients known in the art. Examples of solid excipients include lactose, sucrose, glucose, cornstarch, gelatin, starch, dextrin, calcium phosphate, calcium carbonate, synthetic and natural aluminum silicate, magnesium oxide, dried aluminum hydroxide, magnesium stearate, sodium bicarbonate, and dry yeast. Examples of liquid excipients include water, glycerol, propylene glycol, simple syrups, ethanol, ethylene glycol, polyethylene glycol, sorbitol, etc.

If so-desired, it is possible to add standard additives to the adiponectin expression promoting agent of the present invention. Examples of such additives are citric acid, phosphoric acid, malic acid, and salts thereof and like stabilizers; intense sweeteners such as sucralose, acesulfame potassium, and sucrose, other sugars and the like sweetening agents; alcohols, glycerol and like preservatives; demulcents, diluents, buffers, flavoring agents, and coloring agents.

### (2) Pharmaceutical or food compositions utilizing pharmacological effects of adiponectin

The present invention provides a pharmaceutical or food composition comprising as an active ingredient at least one cyanidin compound selected from the group consisting of the above-mentioned cyanidin and cyanidin glycosides.

The pharmaceutical or food compositions utilize the above-described adiponectin expression promotion effects of the cyanidin compound (cyanidin and cyanidin glycosides). In other words, expression of adiponectin in adipose tissue of human or animal is promoted by using a cyanidin compound, and the increased (activated) pharmacological effects of adiponectin are applied to medicines or foods taken primarily in order to maintain or improve the subject's health.

Examples of cyanidin compounds used as an active ingredient may include those previously mentioned in Item (1), and they may be purified or partially purified. Examples of partially purified cyanidin compounds are cyanidin compound-containing plant extracts and purified fractions thereof.

Examples of known pharmacological effects of adiponectin are anti-obesity, anti-diabetes and anti-arteriosclerosis, and effects of suppressing active oxygen production, etc. (A. H. Berg et al., *Nat. Med.,* 7, (2001): 947-953; T. Yamauchi et al., *Nat. Med.,* 8, (2002): 1288-1295; Iichiro Shimomura et al., *Medical Science Digest,* 28(12), (2002): 17-20; Toshimasa Yamauchi, Takashi Kadowaki, *Experimental Medicine,* 20 (12) (August), (2002): 1762-1767; H. Motoshima et al., *Biochemical and Biophysical Research Communications* 315 (2004) 264-271). Here, obesity, diabetes, arteriosclerosis, and hypertension attributable to obesity, diabetes and arteriosclerosis are examples of pathologies caused by lifestyle-related disease. Therefore, one of favorable medicinal actions of adiponectin is preventing and/or alleviating lifestyle-related diseases. Furthermore, the pharmacological effects of adiponectin encompassed by the present invention include not only the above-explained known medicinal actions but also medicinal actions elucidated in the future.

The scope of the medical composition encompassed by the present invention is not limited by its form or application as long as the medical composition comprises as an active ingredient at least one cyanidin compound selected from the group consisting of the above-described cyanidin and cyanidin glycosides. Preferably, the medical composition of the present invention is orally or parenteraly administered to a human or animal, and is in a form suitable for its administration route. Examples of forms of the medical composition are powders, tablets, emulsions, capsules, granules, chewables, solutions, syrups and like forms suitable for oral administration; and injections, drops, suppositories, patches, creams, ointments, lotions (solutions) and like forms suitable for parenteral administration. It is possible to form the medical composition into these forms by following conventional methods using solid or liquid excipients known in the art.

Food compositions encompassed by the present invention are those taken for the purpose of maintaining good health and/or for improving the subject's health more actively than ordinary foods. Such food compositions can suitably used as health foods intended to prevent, treat and alleviate obesity, diabetes, arteriosclerosis, hypertension or like lifestyle-related diseases, or various pathologies caused by active oxygen.

Examples of food compositions of the present invention include solid, liquid or semisolid food products that use starch, flour, saccharide, syrup, etc., as materials and that are usable as foods as they are. Specific examples thereof are soft white bread, French bread, roll, steamed bread, products baked at a bakery and like breads; udon (thick white wheat noodles), hiyamugi (thin udon), somen (fine white wheat noodles), buckwheat noodles, Chinese noodles, spaghetti, macaroni, bifun (rice vermicelli), harusame (thin potato starch noodles), wonton and like noodles; hard candies (including bonbons, marbles, etc.), soft candies (including caramel, nougat, gummy candies, marshmallows, etc.), drops, taffy and like caramels; hard biscuits, cookies, okaki (rice cracker), senbei (rice cracker) and like baked cakes; fruit juice-containing ice cream, ice lollies and like cold sweets; chewing gum, bubble gum and like gums (stick gums, sugar-coated tablet gums); and other solid food products; separate-type dressings, non-oil dressings, sauces and like flavoring seasonings; soft drinks, carbonated beverages, fruit drinks, vegetable drinks, vegetable and fruit drinks, alcoholic beverages, powder beverages, nutritious drinks and like beverages; consommé soups, potage and like soups; other liquid food products; jelly, yogurt and like desserts; strawberry jam, blueberry jam, preserves and like jams; and other semisolid food products. The food compositions encompassed within the scope of present invention are not limited to those as mentioned above that can be used as foods as they are, and the present invention also encompasses unprocessed food products and semiprocessed food products. Such unprocessed or semiprocessed food products can be prepared into health foods used for the above-described purposes, by mixing, spraying, etc., a cyanidin compound, a cyanidin compound-containing plant extract or a purified fraction thereof in/to foods while processing or in/to end food products. The food compositions encompassed by the present invention include supplements (nutrition supplement foods) in the form of powders, granules, tablets, capsules, drinks, etc.

The dose of the cyanidin compound varies depending on the conditions of the person to be administered, administration method, combination with other medicines and like various factors, while the dose is preferably selected from within the range of 10 to 500 mg per day for an adult with a body weight of 60 kg. The amount of the cyanidin compound contained in the medical or food compositions of the present invention can be adjusted with reference to the above-mentioned preferably daily dosage.

### Examples

Hereunder, the present invention is explained in detail with reference to Examples, although the scope of the invention is not limited to or by these Examples.

### Example 1:

A Wistar male rat (body weight of 160 g) was sacrificed and its epididymis adipose tissues were excised. The epididymis adipose tissues were placed in 0.5% bovine serum albumin (BSA)/30 mM HEPES/200 nM adenosine/Krebs Ringer bicarbonate buffer(BSA-KRBH) containing 1176 units/mL of collagenase (Sigma, type II), and allowed to stand at 37°C for 90 minutes (hereunder, 0.5% BSA/30 mM HEPES/200 nM adenosine/Krebs Ringer bicarbonate buffer is referred to as BSA-KRBH). Adipose cells were isolated from the connective tissue by the above-described operation. The isolated adipose cells were then washed with BSA-KRBH four times, and the thus-obtained adipose cells were suspended in a BSA-KRBH culture medium.

Subsequently, to the thus-obtained suspension, a solution prepared by dissolving cyanidin (Cy; manufactured by Funakoshi Co., Ltd.) or cyanidin 3-*O*-*β*-D-glucoside (hereinafter, referred to as "cyanidin 3-glucoside")(C3G; manufactured by Funakoshi Co., Ltd.) in DMSO was added in such a manner that the final concentration of cyanidin or cyanidin 3-glucoside was 100 µM or 250 µM. The resulting solutions were cultured under 5% CO₂ at 37°C for 24 hours, and the adipose cells were then collected.

Total RNAs were extracted from the thus-obtained adipose cell using an RNA extracting reagent (ISOGEN, manufactured by Nippon Gene Co., Ltd.) by following the instruction manual (http://www.nippongene.jp/pages/products/extraction/isogen/gen01. html). Using the total RNAs as a template, a reverse transcription reaction was conducted at 37°C for 120 minutes, and the RNAs were amplified by 20 cycles of PCR (RT-PCR (Reverse transcription-polymerase chain reaction) method). Specifically, 2.5 µg of total RNAs was subjected to reverse transcription in a buffer solution comprising oligo dT primer, 1.3 mM dNTP (deoxynucleoside triphosphates, GIBCO BRL, Grand Island, NY), 10 mM DTT (dithiothreitol, GIBCO BRL), and 200 U of RNase inhibitor (GIBCO BRL) using 200 U of Moloney murine leukemia virus reverse transcriptase (GIBCO BRL) at 37°C for 120 minutes, then allowed to stand at 94°C for 2 minutes, and the reaction was terminated. The thus-generated cDNAs were amplified in a 0.01% Tween 20/0.2 mM dNTP/2 mM MgCl₂/75 mM Tris buffer (pH 8.8) containing 1 µM 5'-primer(CTCCACCCAAGGAAACTTGT) and 3'-primer(CTGGTCCACATTTTTTTCCT), using 1.5 U of Taq DNA polymerase (MBI Fermentas, Lithuania) by repeating 20 cycles of PCR (each cycle comprised a denaturation step at 94°C for 30 seconds, an annealing step at 58°C for 30 seconds, and an extension reaction step at 72°C for 30 seconds), and then allowed to stand at 72°C for 10 minutes.

The RT-PCR product (502 bp) was applied to a 1% agarose gel with DNA size markers, and subjected to electrophoresis in a Tris-Acetate-EDTA buffer at 100 V for about 30 minutes. After conducting electrophoresis, the gel was dipped in an ethidium bromide solution, shaken for 15 minutes, and then stained. Subsequently, the fluorescence intensity of the gel was measured using a gel analysis program (ATTO Lane Analyzer 10H Software Densitograph provided by Atto Corporation).

Fig. 1 shows the results. Note that, in Fig. 1, the control is epididymis adipose cells to which only DMSO was added. Table 5 shows the adiponectin mRNA levels converted into numerical levels when Cy (cyanidin) or C3G (cyanidin 3-glucoside) was added with the mRNA level of control in Fig. 1 being defined as 1.

**<Table 5>**

| | Control (DMSO) | Cyanidin | | Cyanidin 3-glucoside | |
|---|---|---|---|---|---|
| Concentration | | 100 µM | 250 µM | 100 µM | 250 µM |
| mRNA level | 1.0 | 2.1 | 2.2 | 2.7 | 2.1 |

From the results in Fig. 1 and Table 5, it was confirmed that addition of cyanidin and/or cyanidin 3-glucoside, which is a cyanidin glycoside, increased adiponectin mRNA level in adipose tissue, and therefore expression of adiponectin was promoted.

Adiponectin is known to be effective in alleviating lifestyle-related diseases (i.e., adiponectin has anti-obesity, anti-diabetes, anti-arteriosclerosis and the like effects), and in suppressing production of active oxygen. Therefore, it is believed that cyanidin and cyanidin glycosides can be effectively used for indirectly preventing and/or alleviating the above-mentioned pathologies and various pathologies caused by active oxygen by promoting in vivo expression of adiponectin.

### Example 2 Adiponectin expression promoting agent (1)

Dried cores and seeds of purple corn (10 kg) were placed in a mixed solution (pH 2.3) of 100 L of water and 450 g of sulfuric acid, and allowed to stand overnight at or below room temperature to extract coloring components thereof. After extraction, the thus-obtained extract was filtered using a 60 mesh wire gauze (solid-liquid separation), a filter aid (Radiolite 700 manufactured by Showa Chemical Industry Co., Ltd.) was added to the resulting filtrate in such a manner that the concentration of the filter aid became 2%, and filtrated once more, giving about 100 L of purple corn crude extract.

This crude extract was passed through 10 L of absorption resin (styrene vinylbenzene copolymer: Diaion HP20 manufactured by Mitsubishi Chemical Corporation) under the condition of SV=1 and temperature of 20°C, and the resin was then washed with 30 L (SV=1) of water. Subsequently, 15 L of 30v/v% ethanol /0. 2wt /v% citric acid solution was supplied to the resin (SV=1, temperature at 20°C), and the eluate was collected. The thus-obtained eluate was concentrated under reduced pressure at 40°C or below, obtaining a purple corn extract.

The thus-obtained purple corn extract was passed through 10 L of absorption resin Sepabeads SP207 (manufactured by Mitsubishi Chemical Corporation) under the conditions of SV=1 and a temperature of 20°C, and the obtained resin was washed with 30 L of water (SV=1) and, then, with 10 L of 15v/v% ethanol/0.2wt/v% citric acid solution (SV=1). Subsequently, 20 L of 25v/v% ethanol/0.2wt/v% citric acid solution was passed through the resin (SV=1, temperature of 20°C), and the eluate was collected. The eluate was concentrated under reduced pressure at 40°C or below, and subjected to spray drying, giving 70 g of dark red powder (purple corn extract).

The types and amounts of the cyanidin compounds contained in the thus-obtained powder (purple corn extract) were analyzed by conventional analytical methods (HPLC, MS, NMR, and saccharide analysis after hydrolysis, see H. Aoki et al., *Foods & Food Ingred.* J. Jpn., (2002): 199, 41-45; M. M. Giusti et al., *J. Agric. Food Chem.,* 47, (1999): 4631-4637; and the previously mentioned New *edition, Natural edible colorant).* The analysis found that the powder contained 18.5 wt/wt% of cyanidin 3-glucoside and 3.5 wt/wt% of cyanidin 3-(6-malonyl-glucoside), therefore 22.0 wt/wt% of cyanidin compound in all.

This powder can be provided as the adiponectin expression promoting agent of the present invention as is; after having been purified to increase the content of cyanidin 3-glucoside and cyanidin 3-(6-malonyl-glucoside); or after having separately isolated cyanidin 3-glucoside and cyanidin 3-(6-malonyl-glucoside) from the powder.

### Example 3 Adiponectin expression promoting agent (2)

Using 10 kg of red-kerneled rice instead of the 10 kg of purple corn cores and seeds used in Example 2, cyanidin compounds (cyanidin 3-glucoside and cyanidin 3-(6-malonyl-glucoside) were isolated from red-kerneled rice by following the same manner as in Example 2. The thus-obtained cyanidin compound can be provided as the adiponectin expression promoting agent of the present invention.

### Example 4 Food composition (1)

Using the dark red powder (purple corn extract) prepared in Example 2 as an adiponectin expression promoting agent, a beverage that contained 20% grape juice was prepared. Specifically, materials were mixed in such a manner that the content of each material was as shown below, and after mixed materials were filtered, the filtrate was packed in 250 ml of bottles and sterilized at 80°C for 10 minutes, preparing a beverage that contained 20% grape juice. Note that 250 ml of beverage contained about 50 mg of cyanidin compound (cyanidin 3-glucoside : cyanidin 3-(6-malonyl-glucoside) - 37 : 7 wt/wt)

| | |
|---|---|
| Sucralose | 0.0136 (kg) |
| 5-fold concentrated transparent grape juice | 4.0 |
| Citric acid (crystal) | 0.25 |
| Adiponectin expression promoting agent | 0.10 |
| Grape flavor | 0.10 |
| Fruit flavor | 0.10 |
| Water | Balance |
| Total | 100.00 kg |

### Example 5 Food composition (2)

Sugar-free candies were prepared using the dark red powder (purple corn extract) prepared in Example 2 as the adiponectin expression promoting agent. Specifically, materials were mixed in such a manner that the content of each material was as shown below, melted by heating, and formed into a desired shape, giving sugar-free candies. Note that 5 g of the thus-prepared candy contained about 50 mg of cyanidin compounds (cyanidin 3-glucoside : cyanidin 3-(6-malonyl-glucoside) - 37 : 7 wt/wt).

| | |
|---|---|
| Hydrogenated palatinose | 60.00 (kg) |
| Hydrogenated starch hydrolysate | 59.33 |
| Sucralose | 0.03 |
| Citric acid(anhydrous) | 1.50 |
| Trisodium citrate | 0.07 |
| Adiponectin expression promoting agent | 5.00 |
| Grape flavor | 0.20 |
| Water | 30.00 |
| Amount after being boiled down | 100.00 kg |

### Example 6 Food composition (3)

drinkable jelly was prepared using the dark red powder (purple corn extract) obtained in Example 2 as an adiponectin expression promoting agent. Specifically, materials were mixed in such a manner that the content of each material was as shown below, melted by heating, placed in a container, and sterilized at 85°C for 30 minutes, preparing jellied beverages. Note that 150 g of the jellied beverage contained about 100 mg of cyanidin compounds (cyanidin 3-glucoside : cyanidin 3-(6-malonyl-glucoside) = 37 : 7 wt/wt).

| | |
|---|---|
| Sugar | 5.00 |
| High fructose corn syrup | 15.00 |
| 5-fold concentrated white grape juice | 4.00 |
| Gelling agent (polysaccharide thickener) | 0.20 |
| Gelling agent (gellan gum) | 0.15 |
| Trisodium citrate | 0.10 |
| Calcium lactate | 0.10 |
| Citric acid (anhydrous) | 0.18 |
| Adiponectin expression promoting agent | 0.33 |
| Grape flavor | 0.30 |
| Water | Balance |
| Total | 100.00 kg |

### Example 7 Pharmaceutical composition

Using the dark red powder (purple corn extract) obtained in Example 2 as an adiponectin expression promoting agent, tablets were prepared using a tableting machine after blending materials having the content as shown below. Note that about 50 mg of cyanidin compound (cyanidin 3-glucoside : cyanidin 3-(6-malonyl-glucoside) = 37 : 7 wt/wt) were contained per gram of tablet.

| | |
|---|---|
| Sorbitol | 76.75 (kg) |
| Sucrose fatty acid ester | 0.15 |
| Flavor | 0.1 |
| Adiponectin expression promoting agent | 23.0 |
| Total | 100.0 kg |

### INDUSTRIAL APPLICABILITY

According to the present invention, by promoting and increasing expression of adiponectin, in particular in vivo expression of adiponectin, it is possible to effectively use the pharmacological effects of adiponectin, which living bodies inherently possess. Specifically, using the pharmacological effects of adiponectin, the present invention is effective for preventing or alleviating various pathologies attributable to lifestyle-related diseases such as obesity, diabetes, arteriosclerosis, and the hypertension that is caused by obesity, diabetes and arteriosclerosis, as well as other pathologies caused by active oxygen.

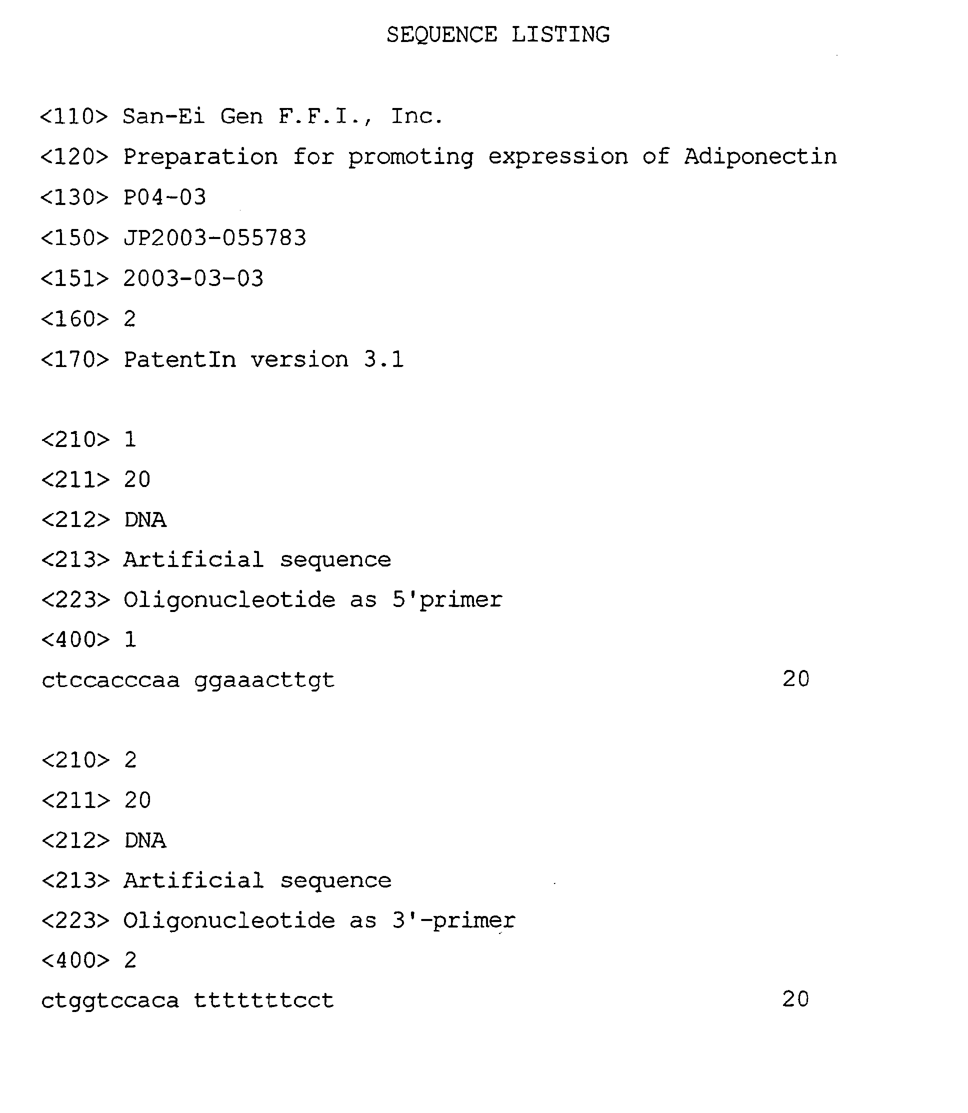

## Claims

1. An adiponectin expression promoting agent comprising as an active ingredient at least one cyanidin compound selected from the group consisting of cyanidin and cyanidin glycosides.

2. An adiponectin expression promoting agent according to Claim 1, wherein the cyanidin glycoside is as shown in General Formula (1) below: 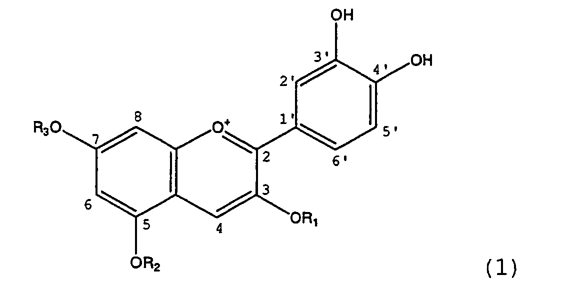 wherein R₁ is a sugar to which an organic acid may be linked, and R₂ and R₃, which may be the same or different, are sugars to which at least one hydrogen atom or organic acid may be linked.

3. An adiponectin expression promoting agent according to Claim 2, wherein the cyanidin glycoside represented by General Formula (1) comprises as the sugar β-D-glucose, β-D-galactose, α-L-rhamnose, β-D-xylose, β-D-arabinose, β-D-glucuronic acid, rutinose, sophorose, gentiobiose, Sambubiose, rhamnose, laminaribiose, gentiotriose, robinobiose, 2^{G}-glucosylrutinose, or 2^{G}-xylosylrutinose.

4. An adiponectin expression promoting agent according to Claim 2, wherein the cyanidin glycoside represented by General Formula (1) comprises, as the sugar, a sugar to which at least one organic acid selected from the group consisting of p-coumaric acid, caffeic acid, ferulic acid, sinapic acid, p-hydroxybenzoic acid, gallic acid, acetic acid, oxalic acid, malonic acid, succinic acid, and malic acid is linked.

5. An adiponectin expression promoting agent according to Claim 2, wherein the cyanidin glycoside represented by General Formula (1) is a cyanidin compound to which a β-D-glucose is directly linked at the 3-position through an oxygen atom with proviso that the β-D-glucose optionally having an organic acid or a further sugar linked thereto.

6. An adiponectin expression promoting agent according to Claim 2, wherein the cyanidin glycoside represented by General Formula (1) is cyanidin 3-*O*-β-D-glucoside.

7. An adiponectin expression promoting agent according to Claim 1, wherein the cyanidin compound is of plant origin.

8. An adiponectin expression promoting agent according to Claim 7, wherein the plant is a purple sweet potato, red cabbage, purple corn, elderberry, strawberry, boysenberry, raspberry, cranberry, blackberry, blueberry, perilla, red-kerneled rice, black-kerneled rice, black soybean, grape, or hibiscus.

9. A pharmaceutical or food composition which is used for an application in which a pharmacological effect of adiponectin is utilized, the pharmaceutical or food composition comprising as an active ingredient at least one cyanidin compound selected from the group consisting of cyanidin and cyanidin glycosides, with the proviso that when the cyanidin compound is cyanidin 3-*O*-β-D-glucoside, a use for anti-obesity or anti-diabetes is excluded.

10. A pharmaceutical or food composition according to Claim 9, wherein the cyanidin glycoside is a cyanidin compound as shown in General Formula (1) below: 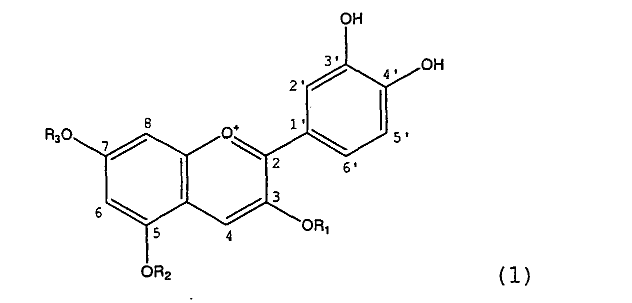 wherein R₁ is a sugar to which an organic acid may be linked, and R₂ and R₃, which may be the same or different, are sugars to which at least one hydrogen atom or organic acid may be linked.

11. A pharmaceutical or food composition according to Claim 10, wherein the cyanidin glycoside represented by General Formula (1) comprises as the sugar β-D-glucose, β-D-galactose, α-L-rhamnose, β-D-xylose, β-D-arabinose, β-D-glucuronic acid, rutinose, sophorose, gentiobiose, Sambubiose, rhamnose, laminaribiose, gentiotriose, robinobiose, 2^{G}-glucosylrutinose, or 2^{G}-xylosylrutinose.

12. A pharmaceutical or food composition according to Claim 10, wherein the cyanidin glycoside represented by General Formula (1) comprises, as the sugar, a sugar to which at least one organic acid selected from the group consisting of p-coumaric acid, caffeic acid, ferulic acid, sinapic acid, p-hydroxybenzoic acid, gallic acid, acetic acid, oxalic acid, malonic acid, succinic acid, and malic acid is linked.

13. A pharmaceutical or food composition according to Claim 10, wherein the cyanidin glycoside represented by General Formula (1) is a cyanidin compound to which a β-D-glucose is directly linked at the 3-position through an oxygen atom with proviso that the β-D-glucose optionally having an organic acid or a further sugar linked thereto.

14. A pharmaceutical or food composition according to Claim 10, wherein the cyanidin glycoside represented by General Formula (1) is cyanidin 3-*O*-β-D-glucoside.

15. A pharmaceutical or food composition according to Claim 9, wherein the cyanidin compound is of plant origin.

16. A pharmaceutical or food composition according to Claim 15, wherein the plant is a purple sweet potato, red cabbage, purple corn, elderberry, strawberry, boysenberry, raspberry, cranberry, blackberry, blueberry, perilla, red-kerneled rice, black-kerneled rice, black soybean, grape, or hibiscus.

17. A pharmaceutical or food composition according to Claim 9, wherein the pharmacological effect of adiponectin is to alleviate a lifestyle-induced disease, and the pharmaceutical or food composition is used for preventing or alleviating a lifestyle-induced disease.

18. A pharmaceutical or food composition according to Claim 9, wherein the adiponectin has at least one pharmacological effect selected from the group consisting of anti-obesity, anti-diabetes, anti-arteriosclerosis, and active oxygen generation reduction, and the pharmaceutical or food composition is used for preventing or alleviating obesity, diabetes, arteriosclerosis, hypertension, or at least one pathology due to active oxygen.
